# EUROPÄISCHE PATENTANMELDUNG

(11) **EP 0 852 951 A1**
(43) Veröffentlichungstag der Anmeldung: **15.07.1998**
(21) Anmeldenummer: 96118489.2
(22) Anmeldetag: 19.11.1996
(51) Int. Cl.: A61K 39/395, A61K 47/18, A61K 47/26

(54) **Stabile lyophilisierte pharmazeutische Zubereitungen von mono- oder polyklonalen Antikörpern**

(71) Anmelder: BOEHRINGER MANNHEIM GMBH, 68298 Mannheim (DE)
(72) Erfinder: Kallmeyer, Georg Dr., 68259 Manheim (DE); Winter, Gerhard Dr., 69221 Dossenheim (DE); Klessen, Christian Dr., 67742 Lauterecken (DE); Woog, Heinrich Dr., 69514 Laudenbach (DE)

(57) **Zusammenfassung**

Die Erfindung betrifft lyophilisierte pharmazeutische Zubereitungen von mono- oder polyklonalen Antikörpern, die einen Zucker oder Aminozucker, eine Aminosäure und ein Tensid als Stabilisierungsmittel enthalten. Außerdem betrifft die Erfindung ein Verfahren zur Herstellung dieser stabilen Lyophilisate sowie die Verwendung eines Zuckers oder Aminozuckers, einer Aminosäure und eines Tensids als Stabilisatoren von antikörperhaltigen therapeutischen oder diagnostischen Mitteln.

## Beschreibung

Die Erfindung betrifft lyophilisierte pharmazeutische Zubereitungen von mono- oder polyklonalen Antikörpern, die einen Zucker oder Aminozucker, eine Aminosäure und ein Tensid als Stabilisierungsmittel enthalten. Außerdem betrifft die Erfindung ein Verfahren zur Herstellung dieser stabilen Lyophilisate sowie die Verwendung eines Zuckers oder Aminozuckers, einer Aminosäure und eines Tensids als Stabilisatoren von antikörperhaltigen therapeutischen oder diagnostischen Mitteln.

Die Herstellung von Immunglobulinen, speziell monoklonalen und polyklonalen Antikörpern, für therapeutische und diagnostische Zwecke ist heute von großer, ständig wachsender Bedeutung.

Der Einsatz von Antikörpern als pharmakologisches Prinzip ist schon lange bekannt und umfaßt zahlreiche Anwendungsmöglichkeiten. So werden Antikörper z.B bei der Tetanus-Prophylaxe, bei der Bekämpfung von pathogenen Mikroorganismen oder der Neutralisation ihrer Toxine, aber auch bei Vergiftungen mit Schlangengiften erfolgreich eingesetzt.

Ist das am Krankheitsmechanismus beteiligte Antigen identifiziert, was bei zahlreichen infektiösen und einigen onkologischen Indikationen für die Antikörpertherapie der Fall ist, macht man sich für die Therapie die Spezifität der Antikörper zunutze.

In klinischen und präklinischen Versuchen werden gegenwärtig Antikörper zur Senkung des Cholesterinspiegels, zur Beeinflussung des Angiotensin/Renin-Systems und bei Autoimmunkrankheiten, wie z.B. Lupus, autoimmune Enzephalitis, Multiple Sklerose, Polyarthritis und autoimmune Myasthenia gravis angewandt.

Von großer therapeutischer Bedeutung ist außerdem ihr Einsatz gegen Intoxikationen durch niedermolekulare Substanzen, wie z.B. den Fab-Fragmenten der Anti-Digoxin-Antikörper beim Einsatz von Intoxikationen durch Digoxin oder den Herzglykosiden Digitoxin und Ouabain. Darüber hinaus werden Antikörper im diagnostischen Bereich zur Identifizierung, Reinigung und Gehaltsbestimmung von Proteinen eingesetzt.

Einen großen Fortschritt für die Bereitstellung von Antikörpern bedeutete die Gentechnologie, die in der zweiten Hälfte der 70er- und in den 80er-Jahren die Produktion von monoklonalen Antikörpern in Zellkulturen revolutionierte.

Um diesen vielseitigen Verwendungsmöglichkeiten gerecht werden zu können, werden lagerstabile pharmazeutische Zubereitungen mono- und polyklonaler Antikörper benötigt. Es gibt eine Reihe von Publikationen, die Flüssigformulierungen oder Lyophilisate spezieller Antikörper zum Inhalt haben.

So sind z.B. Flüssigformulierungen von Antikörpern in EP 280 358, EP 170 983, WO 89/11298, EP 352 500 und J 63 088 197 beschrieben.

Gemäß EP 280 358 wurde der Antikörperlösung zur Stabilisierung gegen bestimmte Hormone Dextran zugesetzt, wodurch eine Stabilität über neun Monate erreicht werden konnte. Nach EP 170 983 wird zur Stabilisierung eines thermolabilen monoklonalen Antikörpers beim Erhitzen hydrolysiertes Ovalbumin eingesetzt, wodurch der Antikörper nach Lagerung bei 45°C noch für 7 Tage noch einsetzbar war. Als weitere Stabilisatoren sind aus JP 63,088,197 für Flüssigrezepturen Polyhydroxyalkohole (z.B. Glycerol, Inositol, Polyvinylalkohol) oder Zucker (z.B. Saccharose und Glukose) bzw. Glycitole (z.B. Sorbitol, Mannitol) bekannt. In WO 89/11298 wird als weitere Möglichkeit zur Flüssigstabilisierung von monoklonalen Antikörpern die Verwendung von Maltose in natriumchloridhaltigem Phosphatpuffer aufgezeigt. EP 352 500 beschreibt zur Flüssigstabilisierung von monoklonalen Antikörpern Polyethylenglykol 4000 und 3-Propiolacton.

Generell sind jedoch Flüssigformulierungen aus Gründen der Lagerstabilität keine optimale Lösung, da die Proteine oder deren Aggregate ausfallen können, die Lösungen verminderten Proteingehalt zeigen und trüb werden.

Dagegen minimiert bei einer Lyophilisatrezeptur die Entfernung des Wassers die Bildung von Abbauprodukten (z.B. durch Deamidierung und Hydrolyse) sowie die Aggregatbildung. Der Restgehalt an Wasser ( 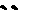 bound water") kann, besonders in Gegenwart von Zuckern, zur Stabilität beitragen (Hsu et al. Dev. Biol. Stand. 1991, **74**, 255-267 und Pikal et al., Dev. Biol. Stand. 1991, **74**, 21-27).

Lyophilisatrezepturen mit speziellen Antikörpern als Wirkstoffe sind ebenfalls aus der Literatur bekannt, geben jedoch keine einheitliche Lehre zum Problem der Stabilisierung. So wird in WO 93/00807 die Stabilisierung von Biomaterialien wie Humanproteinen, Wachstumshormonen, Interleukinen, Interferonen, Enzymen und auch mono- und polyklonalen Antikörpern durch ein Zweikomponentensystem aus Kryoprotektiva (z.B. Polyethylenglykole) und einer Verbindung, die Wasserstoffbrücken zu Proteinen bilden kann, beschrieben Der Nachteil dieser Zubereitungen besteht darin, daß der Zusatz von hochmolekularen Verbindungen wie Polyethylenglykolen im Falle, daß kein Bioabbau erfolgt, zu einer Akkumulation im Körper mit potentiell toxischen Nebenwirkungen führt. Weiterhin können Polymere in Abhängigkeit von der Molmasse bekanntermaßen als Antigene wirken.

Eine Stabilisierung von Lyophilisaten eines einfrierlabilen, monoklonalen Antikörpers über 1 Jahr wird nach J 60146833 A durch Zusatz von Albumin (humanes oder Pferde- oder Rinderalbumin) erreicht. Ebenfalls Humanserumalbumin in Kombination mit einem Kohlenhydrat (z.B. Dextrose, Saccharose oder Maltose) wird zur Stabilisierung eines monoklonalen Antikörpers zur Behandlung von Pseudomonas aeruginosa-Infektionen in EP 303 088 beschrieben.

Humanserumalbumin (in Kombination mit Zuckern und Aminonsäuren) ist auch das Stabilisierungsprinzip von monoklonalen Antikörpern in EP 413 188. In J 01075433 kommt eine Mischung aus Humanserumalbumin, Mannit und Polyethylenglykol zur Stabilisierung eines humanen, monoklonalen Antikörpers als Lyophilisat zum Einsatz. Ein weiteres Beispiel für den Einsatz von Makromolekülen wie z.B. Polyethylenglykolen und Schutzproteinen wie Humanserumalbuminen zur Stabilisierung von Gamma-Globulinen während der Lyophilisation ist in WO 84/00890 gezeigt.

Hagiwara et. al. beschreiben in WO 93/01835 die Stabilisierung eines menschlichen, monoklonalen Antikörpers durch Lyophilisation mit Mannit und Glycin in einer natriumchlorid- und Phosphatpuffer-haltigen Lösung. Es werden stabile Zubereitungen bezüglich Einfrieren, Lyophilisation und Rekonstitution erhalten.

Draber et al. (J. Immun.Methods, **181** 1995, 37-43) konnten eine haltbare Rezeptur von monoklonalen IgM Antikörpern aus der Maus bei 4°C durch Zusatz von Trehalose allein und in Kombination mit Polyethylenglykol 8.000 erzielen. Allerdings sind die Antikörper bei 50 °C nur 14 Tage stabil. Mit anderen Mono- oder Disacchariden wie z.B. Saccharose, Maltose, Lactose oder Galactose allein gelingt eine Stabilisierung dieser Antikörper nicht.

In WO 89/11297 wird ein monoklonaler Antikörper aus der Maus mit einem Kohlenhydrat, nämlich Maltose, und einem Puffer im sauren Bereich (Acetatpuffer) in ein stabiles Lyophilisat überführt. Der Nachteil ist hier die Beschränkung der Pufferung auf den sauren Bereich.

Polymere Gelatine als Einfrierschutz und Stabilisierungsmittel in einem Lyophilisat wird in WO 92/15 331 verwendet. Die Stabilisierung erfolgt auch in Kombination mit einer Carbonsäure (z.B. Citronensäure) oder deren Salz sowie mit einem primären, sekundären oder tertiären Alkohol oder einer Aminosäure in einem pH-Bereich von 6,8 bis 8,1.

In einer ganzen Reihe der vorstehend genannten Druckschriften werden als Stabilisatoren pharmazeutische Zusatz- oder Hilfsstoffe vorgeschlagen, die aus medizinischer Sicht nicht unbedenklich sind. So stellen Polymere (wie PEG oder Gelatine) und Proteine (wie Serumalbumine) auf Grund ihrer Herkunft und ihrer physikalisch-chemischen Eigenschaften ein gewisses Risiko dar und können allergische Reaktionen bis hin zum anaphylaktischen Schock auslösen. Proteine menschlichen oder tierischen Ursprungs sowie aus Zellkulturen gewonnene Proteine tragen ein Restrisiko viraler Verunreinigungen. Aber auch andere, analytisch schwer nachweisbare proteinartige Verunreingungen können wegen ihrer Eigenschaften immunologische Reaktionen beim Menschen hervorrufen.

Der Zusatz polymerer Verbindungen, wie z.B. von Polyethylenglykolen (PEG) oder Gelatine kann im Fall, daß kein Bioabbau erfolgt, zu einer Akkumulation im Körper mit potentiell toxischen Nebenwirkungen führen. In Abhängigkeit von der Molmasse können Polymere auch antigene Eigenschaften aufweisen. Auch ist die Reinheit von Polymeren aufgrund der zur Herstellung verwendeten Katalysatoren oder des Vorhandenseins von Monomeren und anderen Polymerbruchstücken schwierig zu gewährleisten. Der Einsatz von Polymeren bei pharmazeutischen Darreichungsformen, insbesondere bei subkutan applizierbaren Arzneiformen, ist zu vermeiden, wenn eine Stabilisierung auf andere Art und Weise möglich ist.

Dagegen gewährleistet die Verwendung von Zuckern allein ohne weitere Zusatzstoffe nicht in allen Fällen die entsprechende Schutzwirkung beim Lyophilisieren der Antikörper.

Der Erfindung lag deshalb die Aufgabe zugrunde, eine stabile pharmazeutische Zubereitung mono- oder polyklonaler Antikörper bereitzustellen, insbesondere auch von einfrierempfindlichen Antikörpern. Die Zubereitung soll keine toxikologisch bedenklichen polymeren oder proteinartigen Hilfsstoffe enthalten, physiologisch verträglich, einfach zusammengesetzt und exakt dosierbar sein, sowie stabil sowohl gegenüber Einfrier- und Auftauvorgängen als auch bei längerer Lagerung als Lyophilisat und bei höheren Temperaturen.

Die Aufgabe der Erfindung wird durch eine lyophilisierte pharmazeutische Zubereitung von mono- oder polyklonalen Antikörpern gelöst die als Zusatzstoffe einen Zucker oder einen Aminozucker, eine Aminosäure und ein Tensid enthalten. Überraschenderweise bewirkt diese Kombination an Zusatzstoffen einen Einfrierschutz und ermöglicht so die Lyophilisation bei Temperaturen bis zu -45°C, ohne die Stabilität der Antikörper negativ zu beeinträchtigen. Daneben sind die Lyophilisate mit der erfindungsgemäßen Kombination an Zusatzstoffen auch bei höheren Temperaturen langzeitstabil und lagerstabil. Sie zeigen insbesondere im Vergleich zu herkömmlichen Rezepturen nach Rekonstitution mit Wasser keine Partikelbildungen.

Die erfindungsgemäßen Zubereitungen besitzen außerdem den Vorteil, daß sie im wesentlichen frei von proteinartigen oder polymeren Hilfsstoffen sind, deren Verwendung aus medizinischer Sicht nicht unproblematisch sein kann. Aufgrund der Tatsache, daß nunmehr durch Auflösen von Lyophilisaten erhaltene, flüssige antikörperhaltige therapeutische oder diagnostische Mittel mit einem pH-Wert von etwa 5 - 8, vorzugsweise mit einem pH-Wert 6,0 - 7,4 (pH-Wert des Blutes 7,2 - 7,4), hergestellt werden können, besitzen sie ferner den Vorteil, gut verträglich und weitgehend schmerzfrei applizierbar zu sein. Dies ist vor allem bei subkutaner Gabe wesentlich, da hier leichter Unverträglichkeiten entstehen als bei intravenöser Gabe.

Die erfindungsgemäßen Zubereitungen lassen sich generell in klinisch relevanten Konzentrationsbereichen des Antikörpers, beispielsweise von bis zu 20 mg/ml, bevorzugt bis zu 10 mg/ml, herstellen. Bevorzugte Konzentrationsbereiche liegen bei Konzentrationen ab 0,01 mg/ml, insbesondere ab 0,05 bzw. 0,1 mg/ml. Insbesondere werden beispielsweise Konzentrationsbereiche von 0,05 - 10 mg/ml oder 0,1 - 5 mg/ml, beispielsweise etwa 5, 8 oder 10 mg/ml verwendet. Die Injektionsvolumina der verwendeten Lösungen liegen bei weniger als 2 ml, vorzugsweise bei etwa 1 ml im Falle von subcutanen oder intravenösen Injektionen. Kleine Injektionsvolumen sind bei subkutaner Applikation besonders vorteilhaft, da sie nur geringe mechanische Reize im Unterhautgewebe hervorrufen. Grundsätzlich sind die Lösungen auch als Zusätze zu Infusionslösungen oder als Infusionslösungen direkt geeignet. Für den Fall, daß sie als Zusätze zu Infüsionslösungen verwendet werden, liegt die Konzentration der Antikörper bei höheren Werten, beispielsweise bei bis zu 10 mg/ml. Diese konzentrierten Lösungen der Antkörper werden dann üblichen Infusionslösungen zugesetzt, so daß die Konzentration des Antikörpers in der zu applizierenden Infusionslösung im therapeutisch relevanten Bereich liegt. Dieser Bereich beträgt normalerweise 0,001 - 0,5 mg/ml.

Die pharmazeutischen Einzeldarreichungsformen können entweder als gebrauchsfertige Infusions- oder Injektionslösungen oder auch als Lyophilisate vorliegen. Falls Lyophilisate eingesetzt werden, so enthalten die Einzeldosisbehälter, beispielsweise Glasampullen mit einem Volumen von 10 ml, den Antikörper in Mengen von 0,1 - 500 mg, vorzugsweise 10 - 100 mg in Abhängigkeit von der jeweiligen therapeutisch relevanten Dosis des Antikörpers. Das Lyophilisat enthält gegebenenfalls weitere pharmazeutisch übliche Hilfsstoffe. Das Lyophilisat wird mit einer entsprechenden Menge an Rekonstitutionslösung aufgelöst und kann dann entweder als Injektionslösung direkt oder als Zusatz zu einer Infusionslösung eingesetzt werden. Im Fall der Verwendung als Zusatz zu Infusionslösungen wird das Lyophilisat in der Regel mit etwa 10 ml einer Rekonstitutionslösung aufgelöst und zu einer physiologischen Kochsalzlösung (0,9 % NaCl) von 250 ml zugegeben. Die so resultierende Infusionslösung wird dann üblicherweise innerhalb von etwa 30 Minuten dem Patienten verabreicht.

Die erfindungsgemäß eingesetzten Zucker können Mono-, Di- oder Trisaccharide sein. Als Monosaccharide kommen Glucose, Mannose, Galactose, Fructose und Sorbose in Frage, als Disaccharide Saccharose, Lactose, Maltose, Trehalose und als Trisaccharid kommt vorzugsweise Raffinose in Frage. Besonders bevorzugt werden erfindungsgemäß Saccharose, Lactose, Maltose, Raffinose oder Trehalose eingesetzt. Anstatt von Maltose können auch die stereoisomeren Disaccharide Cellobiose, Gentiobiose oder Isomaltose eingesetzt werden.

Als Aminozucker werden generell solche Monosaccharide bezeichnet und eingesetzt, die anstelle einer Hydroxygruppe eine Amino- oder eine acylierte Aminogruppe besitzen Erfindungsgemäß besonders bevorzugt sind hierfür Glucosamin, N-Methylglucosamin, Galactosamin und Neuraminsäure. Der Zuckergehalt oder Aminozuckergehalt beträgt beispielsweise bis zu 2000 mg, vorzugsweise bis zu 1000 mg, insbesondere bis zu 800 bzw. bis zu 500 mg pro Einzeldarreichungsform. Als untere Grenze für den Zuckergehalt kommen beispielsweise Mengen ab 10, 50 bzw 100 mg in Frage. Bevorzugte Bereiche sind 200 - 1000 mg, insbesondere 400 - 800 mg. Die hier genannten Mengenangaben pro Einzeldarreichungsform beziehen sich Einzeldarreichungsformen, die als Lyophilisate in den Handel kommen. Derartige Lyophilisate sind vorzugsweise in Injektionsflaschen mit einem Volume von 10 ml abgefüllt. Nach Aufllösung der Lyophilisate mit einer Rekonstitutionslösung von 10 ml werden flüssige Darreichungsformen erhalten, die direkt verabreicht werden können. Die Zuckerkonzentration in diesen Injektionslösungen beträgt bis zu 200 mg/ml, vorzugsweise bis zu 100 mg/ml, basierend auf den eingangs genannten Mengenangaben der verwendeten Zucker.

Als erfindungsgemäß verwendete Aminosäuren kommen basische Aminosauren in Frage, wie beispielsweise Arginin, Lysin, Histidin, Ornithin u.a., die vorzugsweise auch als Aminosäurephosphate eingesetzt werden können. Daneben können auch saure Aminosäuren wie z.B. Glutaminsäure und Asparaginsäure oder neutrale Aminosäuren wie z.B. Isoleucin, Leucin und Alanin bzw. aromatische Aminosäuren wie z.B. Phenylalanin, Tyrosin oder Tryptophan Verwendung finden. Der Aminosäuregehalt in den erfindungsgemäßen wäßrigen Zubereitungen beträgt bis zu 100 mg/ml, bevorzugt bis zu 50 mg/ml oder bis zu 30 mg/ml. Als untere Grenze kommen beispielsweise Konzentrationen ab 1, 5 oder 10 mg/ml in Frage. Bevorzugte Konzentrationen liegen beispielsweise im Bereich von 3 - 30 mg/ml bzw. 10 - 25 mg/ml. Für den Fall, daß die entsprechenden Darreichungsformen als Lyophilisate in den Handel kommen, werden diese Lyophilisate vorzugsweise in Injektionsflaschen (Volumen von beispielsweise 10 ml) zur Verfügung gestellt. Derartige Einzeldarreichungsformen enthalten die Aminosäuren in einer Menge von bis zu 1000 mg, bevorzugt bis zu 500 mg oder bis zu 300 mg.

Als Tenside kommen alle in pharmazeutischen Zubereitungen üblicherweise eingesetzten Tenside in Frage, vorzugsweise Polysorbate und Polyoxyethylen-polyoxypropylen-Polymere. Es sind niedrige Tensidmengen von 0,05 bis 0,5 mg/ml, vorzugsweise 0,1 mg/ml, ausreichend zur Stabilisierung der Antikörper. In den oben erwähnten Einzeldarreichungsformen beträgt die Menge der Tenside 0,5 - 5 mg im Falle eines Lyophilisates, das in einer Injektionsflasche von 10 ml abgefüllt ist.

Die mittels der genannten Zusatzstoffe erfolgte Stabilisierung von Antikörpern bezieht sich prinzipiell auf alle bekannten monoklonalen und polyklonalen Antikörper und deren Fab-Fragmente. Das Molekulargewicht der Antikörper beträgt 50 kDa - 200 kDa pro Monomereinheit, insbesondere liegt das Molekulargewicht bei etwa 80 - 150 kDa. Insbesondere können erfindungsgemäß Antikörper gegen das Hepatitis-B-Virus, gegen AIDS-Viren, Cytomegalie-Viren, Meningoenzephalitis-Viren (FSME), Röteln-Viren, Masern-Viren, Tollwuterreger, Pseudomonas aeruginosa-Bakterien, Varizella-Zoster-Viren, Tetanuserreger oder Diephterieerreger stabilisiert werden. Die Antikörperkonzentration kann vorzugsweise bis zu 8 mg/ml betragen. Vorzugsweise beträgt sie beispielsweise 0,05 - 2 mg/ml. Die Menge an Antikörper in der Einzeldarreichungsform, beispielsweise in einem Lyophilisat in einer Injektionsflasche von 10 ml, beträgt bis zu 50 mg, vorzugsweise bis zu 20 mg oder 10 mg.

Neben den genannten Zusatzstoffen Zucker, Aminosäure und Tensid können die erfindungsgemäßen Lyophilisate physiologisch verträgliche Hilfsstoffe aus der Gruppe der Säuren, Basen, Puffer oder Isotonisierungsmittel zur Einstellung des pH-Wertes auf 5 bis 8, vorzugsweise 6,0 bis 7,4 enthalten. Die Pufferkapazität der Präparate wird so eingestellt, daß beim Auflösen der Lyophilisate mit üblichen Rekonstitutionslösungen, wie beispielsweise Wasser für Injektionszwecke, die Pufferkonzentration im Bereich zwischen 10 - 20 mMol/l, bevorzugt bei etwa 15 mMol/l, liegt.

Die Reihenfolge der Zugabe der verschiedenen Hilfsstoffe oder des Antikörpers ist weitgehend unabhängig hinsichtlich des Herstellungsverfahrens und liegt im Ermessen des Fachmannes. Der gewünschte pH-Wert der Lösung wird durch Zugabe von Basen, wie beispielsweise von Alkalihydroxiden, Erdalkalihydroxiden oder Ammoniumhydroxid eingestellt. Vorzugsweise wird hierzu Kaliumhydroxid verwendet. Die Einstellung des gewünschten pH-Wertes ist prinzipiell durch Zugabe von basischen Lösungen möglich. In diesem Sinne kommen allgemein Salze von starken Basen mit schwachen Säuren in Frage, wie z. B. Natriumacetat, Natriumcitrat, Di-Natrium- bzw. Di-Kaliumhydrogenphosphat oder Natriumcarbonat. Für den Fall, daß die pharmazeutische Hilfsstofflösung einen basischen pH-Wert aufweist, erfolgt die Einstellung durch Titration mit Säure, bis der gewünschte pH-Bereich erreicht ist. Als Säuren kommen physiologisch verträgliche anorganische oder organische Säuren in Frage, wie beispielsweise Salzsäure, Phosphorsaure, Essigsäure, Zitronensäure oder allgemein übliche Lösungen von Substanzen, die einen sauren pH-Wert besitzen. Bevorzugte Substanzen sind in diesem Sinne Salze von starken Säuren mit schwachen Basen, wie z. B. Natriumdihydrogenphosphat oder Kaliumdihydrogenphosphat. Bevorzugt wird der pH-Wert der Lösung mit Phosphorsäure oder einer wässrigen Kaliumhydroxidlösung eingestellt.

Zur Herstellung gut verträglicher parenteraler Arzneiformen ist der Zusatz von isotonisierenden Hilfsstoffen zweckmäßig, wenn nicht durch die osmotischen Eigenschaften des Antikörpers und der zur Stabilisierung eingesetzten Zusatzstoffe bereits Isotonie erreicht werden kann. Dazu werden vor allem nicht-ionisierte, gut verträgliche Hilfsstoffe eingesetzt. Der Zusatz von Salzen, z.B. NaCl, sollte jedoch nur in geringen Mengen erfolgen, insbesondere sollte vorzugsweise ein Wert von 30 mMol/l in der fertig applizierbaren Injektions- oder Infusionslösung nicht überschritten werden.

Außerdem können die pharmazeutischen Zubereitungen weitere übliche Hilfs- oder Zusatzstoffe enthalten. Es können Antioxidanzien, wie beispielsweise Glutathion oder Ascorbinsäure oder ähnliche Substanzen zugesetzt werden.

Zur Herstellung der Lyophilisate werden zunächst die wäßrigen pharmazeutischen Lösungen, die den Antikörper enthalten, hergestellt. Bevorzugt wird eine gepufferte natriumchloridhaltige Antikörperlösung hergestellt. Dieser Antikörper-Lösung wird eine wäßrige Lösung mit den Zusatzstoffen Zucker, Aminosäure und Tensid beigemischt, wobei der pH-Wert mit einer Säure oder Base auf 5 bis 8 eingestellt wird. Der Zusatz von Phosphorsäure bzw. Phosphatsalzen und Natriumchlorid erfolgt in solchen Mengen, so daß die zuvor definierten Konzentrationen erhalten werden. Anschließend erfolgt die Sterilfiltration und Lyophilisation der so hergestellten Lösung.

Mit der Erfindung können ohne Verschlechterung der Qualität auch einfrierempfindliche Antikörper enthaltende instabile, wäßrige Lösungen mittels Gefriertrocknung in, auch bei hohen Temperaturen, stabile Zubereitungen überführt werden.

Die erfindungsgemäßen Lyophilisate haben außerdem den Vorteil, daß sie neben Vermeidung einer Schädigung der Antikörper beim Einfrieren auch nach einer Langzeitlagerung bei 50 °C keine Abnahme im Antikörpergehalt und keine Aggregatbildung oder ein Ausflocken zeigen. Sie sind somit hinsichtlich Antikörpergehalt und Reinheit stabil Partikelbildungen werden verhindert, was sich in den geringen Trübungswerten nach Rekonstitution der Lyophilisate mit Wasser für Injektionszwecke zeigt.

Im folgenden wird die Erfindung anhand von Ausführungsbeispielen näher erläutert:

Die Beispiele 1 bis 10 zeigen, in welcher Weise die erfindungsgemäßen Lyophilisate formuliert, hergestellt und hinsichtlich Antikörperstabilität untersucht werden können.

Vergleichende Untersuchungen ohne Hilfsstoffe oder mit Saccharose allein oder mit Mannit als Ersatz der Zuckerkomponente oder mit der Aminosäurekomponente allein oder nur Zucker- und Aminosäurekomponente ohne Tensid zeigen, daß die Auswahl der erfindungsgemäßen Kombination der Zusatzstoffe aussschlaggebend für das Erreichen einer stabilen Rezeptur ist. Sowohl Saccharose allein, Aminosäure allein oder beide Komponenten ohne Tensid führen zu instabilen Rezepturen.

Die erfindungsgemäßen Rezepturen sind gegen Einfrieren unempfindlich, und auf die als toxisch anzusehenden Polymere oder Proteine wie Polyethylenglykole, Gelatine, Serumalbumine kann vollständig verzichtet werden. Bei den Tensiden handelt es sich nur um relativ geringe Mengen an physiologisch gut verträglichen Tensiden.

Bei dem in den folgenden Ausführungsbeispielen eingesetzten Antikörper gegen HBV handelt es sich um einen rekombinanten, humanen, monoklonalen Antikörper (MAK) aus einer murinen Zelle. Er besitzt ein Molekulargewicht von etwa 147 kDa und ist gegen das Hepatitis B-Oberflächenantigen (HBsAg) des Hepatitis B-Virus gerichtet. Der monoklonale Antikörper erkennt die a-Determinante des HBsAg, die in nahezu allen bekannten Varanten des Virus konstant ist. Dieser Antikörper kann beispielsweise für folgende medizinische Indikationen eingesetzt werden: Behandlung der chronischen Hepatitis, für die bisher noch keine befriedigende Behandlunsgtmöglichkeit existiert, Behandlung der passiven Immunprophylaxe in HBsAg-positiven Lebertransplantationspatienten. In Mittel- und Nordeuropa und den USA sind bis zu 2 % der Bevölkerung Träger des Hepatities B-Virus, in Südeuropa bis zu 3 %, in Afrika und Fernaost sind es 10 - 15 %. Als Konsequenz dieser chronischen Infektion ist die Entwicklung eines hepatozellulären Carcinoms um das 100-fache erhöht, 40 % der Virusträger sterben als Folge dieser Infektion.

Beispiel 1 zeigt das Verhalten einer wäßrigen, phosphatpuffer- und natriumchloridhaltigen Lösung eines monoklonalen Antikörpers gegen Hepatitis B Virus (MAK HBV) bei pH=5, pH=6,5 und pH=8 nach Einfrieren und Auftauen. Es zeigt, daß Einfrieren und Auftauen den monoklonalen Antikörper schädigt.

Beispiel 2 demonstriert die Möglichkeit der Stabilisierung einer erfindungsgemäßen Zubereitung mit Saccharose oder Maltose oder einem Aminozucker (N-Methylglucosamin oder Galactosamin) und Argininphosphat und Tween 20 mit einer Konzentration des Antikörpers von 2 mg/ml, d.h. 2 mg im Lyophilisat.

In Beispiel 2a ist dieselbe Zubereitung wie in Beispiel 2 gezeigt, nur mit der Antikörperkonzentration 8 mg/ml. An den Beispielen 2 und 2a wird sichtbar, daß durch die Kombination der erwähnten Hilfsstoffe nicht nur die Schädigung des Antikörpers beim Einfrieren vermieden wird, sondern auch ein positiver Einfluß auf die Stabilität bei Langzeitlagerung ausgeübt wird.

Beispiel 3 erläutert die Notwendigkeit von Aminosäuren und Tensid in der erfindungsgemäßen Zubereitung. Die Verwendung von Saccharose als Gerüstbildner allein führt zu einem instabilen Lyophilisat.

In Beispiel 4 wird die Variation der Aminosäurekomponente beschrieben. Es zeigt sich, daß sowohl die Variation der basischen Aminosäuren in Form von Arginin oder Ornithin als auch der Ersatz der basischen Aminosäure durch eine neutrale Aminosäure, wie z.B. durch Leucin oder durch eine saure Aminosäure, wie z. B. durch Asparaginsäure, zu einer lagerstabilen Zubereitung führt.

In Beispiel 5 wird die Lyophilisation einer Rezeptur mit Saccharose, Arginin und Tween 20 sowie Kaliumphosphatpuffer und Natriumchlorid bei verschiedenen pH-Werten (pH 5, pH 6,5 und pH 8) verglichen. Die erhaltenen Daten zeigen, daß eine Lyophilisation innerhalb dieses pH-Bereiches ohne Beeinträchtigung der Stabilität möglich ist.

Ersetzt man das erwähnte Tensid Tween 20 durch einen Vertreter der oberflächenaktiven Verbindungsklasse Polyoxyethylenpolyoxypropylen-Polymere (Handelsname Pluronic®) wie in Beispiel 6, ergibt sich ebenfalls eine hinreichende Stabilität der erfindungsgemäßen Zubereitung.

Beispiel 7 demonstriert die Instabilität einer Rezeptur mit Mannit als Gerüstbildner als Ersatz für Saccharose, Maltose oder den Aminozucker (siehe Beispiel 2).

Läßt man in der Rezeptur den Zucker und das Tensid weg, wie in Beispiel 8 gezeigt, wird die Zubereitung instabil.

Eine Kombination aus Zucker (z.B. Saccharose) und Aminosäuren ohne Tensid in Beispiel 9 zeigt zwar bei den Parametern Gehalt und Aggregate gute Ergebnisse, die Turbidität war jedoch wesentlich erhöht im Vergleich zu den erfindungsgemäßen Rezepturen mit Zucker, Aminosäure und Tensid.

Beispiel 10 zeigt, daß auch andere monoklonale Antikörper mit einer Kombination des Zuckers, Aminosäure und Tensid stabilisiert werden können. Der Antikörper Anti-L-Selektin ist beispielsweise in einer Konzentration von 7 mg im Lyophilisat stabil. Die Lyophilisation erfolgt ausgehend von einem Volumen von 1 ml einer wässrigen Lösung.

### Untersuchungsmethoden zur Stabilitätsbestimmung

Die lyophilisierten Zubereitungen wurden unter Lichtauschluß bei definierten Lagerbedingungen gelagen und danach analysiert. Für die Analysen wurden folgende Testmethoden verwendet.
OD 280: Optische Dichte bei 280 nm. Photometrische Bestimmung des Proteingehaltes, die UV-Absorption erfolgt aufgrund von Seitenkettenchromophoren wie Tryptophan-Tyrosin- und Phenylalaninreste. Spezifikation: 95-105%
SE-HPLC: Size-exclusion-Hochleistungschromatographie zur Bestimmung von Aggregaten. Spezifikation: max. 2%.
Trübungsmessung: Nach Rekonstitution des Lyophilisates wurde die Messung an der unverdünnten Antikörperlösung in einem geeigneten Trübungsphotometer durchgeführt. Spezifikation: max. 6 Trübungseinheiten.

### Beispiel 1:

Es wird eine wäßrige, phosphatpuffer- und natriumchloridhaltige Stammlösung des oben beschriebenen MAK gegen HBV hergestellt und untersucht. Die Konzentration des MAK liegt bei etwa 15 mg/ml.

Die Tabelle 1a zeigt zum einen die Einfrierlabilität der monoklonalen Antikörperlösung bei verschiedenen pH-Werten bei -20°C, wo sich bereits nach 4 Wochen ein Abfall des Proteingehaltes auf 92,1 bzw. 94,2 bzw. 94,0 % ergibt. Ebenso wird ein Abfall des Proteingehaltes bei Lagerung bei 25°C beobachtet. Unter der Lagerbedingung 4-8°C im Kühlschrank ist der Antikörper über 9 Monate hinreichend stabil.

Die Tabellen 1b-1d zeigen die Stabilitätsdaten der hergestellten monoklonalen Antikörperlösung bei pH-Werten 5, 6,5 und 8 bei -20°C, 4-8°C und 25°C. Auch hier zeigt sich, daß nur eine Lagerung bei 4-8°C akzeptabel ist.

**Tabelle 1a**

| Veränderung des Antikörpergehaltes in der Wirkstofflösung (10 mM Kaliumphosphatpuffer, 30 mM Natriumchlorid, Wasser für Injektionszwecke) | | | | | | | | | |
|---|---|---|---|---|---|---|---|---|---|
| | **pH 5** | | | **pH 6,5** | | | **pH 8** | | |
| **Zeitpunkt** | **-20°C** | **4-8°C** | **25°C** | **-20°C** | **4-8°C** | **25°C** | **-20°C** | **4-8°C** | **25°C** |
| Start | | > 99 | | | > 99 | | | > 99 | |
| 4 Wochen | 92,1 | > 99 | > 99 | 94,2 | > 99 | > 99 | 94,0 | > 99 | > 99 |
| 13 Wochen | 78,9 | > 99 | 97,2 | 81,2 | > 99 | 98,1 | 77,8 | > 99 | 96,1 |
| 6 Monate | 61,2 | > 99 | 94,1 | 69,9 | > 99 | 94,4 | 65,8 | > 99 | 91,9 |
| 9 Monate | 47,8 | > 99 | 88,7 | 55,6 | > 99 | 90,2 | 51,0 | > 99 | 84,3 |

Alle Angaben in %. Die Proteinbestimmung erfolgte mittels Messung der Absorption bei 280 nm (OD 280).

**Tabelle 1b**

| Aggregatbildung und Trübungswerte der Antikörper-Wirkstofflösung, pH = 5 | | | | | | |
|---|---|---|---|---|---|---|
| Zeitpunkte | -20°C | | 4-8°C | | 25°C | |
| | Aggregate | Trübung | Aggregate | Trübung | Aggregate | Trübung |
| Start | n.n | 1,5 | n.n. | 1,5 | n.n | 1,5 |
| 4 Wochen | Aggregate | Ausflock. | n.n. | 1,5 | 0,7 % | 1,5 |
| 13 Wochen | Aggregate | Ausflock. | 0,2 % | 1,8 | 1,9 % | 1,8 |
| 6 Monate | Aggregate | Ausflock. | 0,3 % | 1,9 | Aggregate | 9,9 |
| 9 Monate | Aggregate | Ausflock. | 0,6 % | 2,1 | Aggregate | 10,9 |
| n.n. = nicht nachweisbar | | | | | | |

**Tabelle 1c**

| Aggregatbildung und Trübungswerte der Antikörper-Wirkstofflösung, pH = 6,5 | | | | | | |
|---|---|---|---|---|---|---|
| Zeitpunkte | -20°C | | 4-8°C | | 25°C | |
| | Aggregate | Trübung | Aggregate | Trübung | Aggregate | Trübung |
| Start | n.n | 1,2 | n.n | 1,2 | n.n. | 1,2 |
| 4 Wochen | Aggregate | Ausflock. | n.n. | 1,3 | 0,5 % | 1,4 |
| 13 Wochen | Aggregate | Ausflock. | 0,2 % | 1,4 | 1,8 % | 1,7 |
| 6 Monate | Aggregate | Ausflock. | 0,3 % | 1,9 | 4,9 % | Ausflock. |
| 9 Monate | Aggregate | Ausflock. | 0,6 % | 2,1 | 9,3 % | Ausflock. |

**Tabelle 1d**

| Aggregatbildung und Trübungswerte der Antikörper-Wirkstofflösung, pH = 8 | | | | | | |
|---|---|---|---|---|---|---|
| Zeitpunkte | -20°C | | 4-8°C | | 25°C | |
| | Aggregate | Trübung | Aggregate | Trübung | Aggregate | Trübung |
| Start | n.n | 1,4 | n.n | 1,4 | n.n | 1,4 |
| 4 Wochen | 2,0 % | Ausflock. | 0,3 % | 1,5 | 0,74 % | 1,7 |
| 13 Wochen | 2,8 % | Ausflock. | 0,5 % | 1,8 | 1,95 % | 2,1 |
| 6 Monate | 3,7 % | Ausflock. | 0,6 % | 1,9 | 3,0 % | Ausflock. |
| 9 Monate | 5,4 % | Ausflock. | 0,8 % | 2,1 | 4,3 % | Ausflock. |

Aggregate in % mit SE-HPLC, Trübung in Trübungseinheiten (Turbidität) mit Trübungsphotometer

### Beispiel 2:

Wäßrige Lösungen der nachfolgend genannten Zucker bzw. Aminozucker Sacchar (Rezeptur 1), Maltose (Rezeptur 2) und N-Methylglucosamin (Rezeptur 3) Argininphosphat-Puffer und Tween 20 als Tensid wurden mit einer Lösung des mo klonalen Antikörpers gegen HBV gemäß Beispiel 1 versetzt. Eine Übersicht Rezeptur ist in Beispiel 2a dargestellt. Die Endkonzentration des MAK beträgt 2 mg/ml.

Die Lösungen wurden nach Einstellung des pH-Wertes mit Phosphorsaure auf 6,5 sterilfiltriert (0,22µm Membranfilter) und in sterile und entpyrogenisierte Injektionsflaschen aus Glas (hydrolytische Klasse I) abgefüllt (Füllvolumen 1 ml) und lyophilisiert. Nach Lyophilisation wurden die Injektionsflaschen mit Stickstoff belüftet, mit Stopfen automatisch in der Gefriertrocknungskammer verschlossen, danach verbördelt.

Die Lagerung der verbördelten Injektionsflaschen erfolgte unter Lichtausschluß über 4 und 13 Wochen bei verschiedenen Temperaturen. Nach diesen Zeitpunkten wurde die Stabilität der Lyophilisate mit den beschriebenen Untersuchungsmethoden untersucht.

**Tabelle 2a**

| Lagerung bei 25°C | | | | | | |
|---|---|---|---|---|---|---|
| | Lagerung 4 Wochen bei 25°C | | | Lagerung 13 Wochen bei 25°C | | |
| | I | II | III | I | II | III |
| Rez. 1 Saccharose | 100 | n.n. | 1,7 | 100 | n.n. | 1,6 |
| Rez. 2 Maltose | 100 | n.n. | 1,6 | 100 | n.n. | 1,8 |
| Rez. 3 N-Methylglucosamin | 100 | n.n. | 1,8 | 100 | n.n | 1,5 |

**Tabelle 2b**

| Lagerung bei 50°C | | | | | | |
|---|---|---|---|---|---|---|
| | Lagerung 4 Wochen bei 50°C | | | Lagerung 13 Wochen bei 50°C | | |
| | I | II | III | I | II | III |
| Rez. 1 Saccharose | >99 | n.n. | 2,0 | >99 | n.n | 2,0 |
| Rez. 2 Maltose | >99 | n.n. | 1,9 | >99 | n.n | 2,1 |
| Rez. 3 N-Methylglucosamin | >99 | n.n | 1,7 | >99 | n.n. | 2,0 |
| Legende: I Proteingehalt in % mit OD 280 II Aggregate in % mit SE-HPLC III Trübung der rekonstituierten Lösung in Turbiditätseinheiten (dimensionslose Zahl) n.n. nicht nachweisbar (auch in allen weiteren Tabellen so benutzt) | | | | | | |

### Beispiel 2 a

In Beispiel 2 a ist die Rezeptur 1 aus Beispiel 2 mit 8 mg/ml Antikörper (= Rezeptur 1a) dargestellt. Es zeigt sich, daß in dieser Rezeptur auch höhere Konzentrationen bis zu 8 mg/1 ml Antikörper hinreichend stabil sind.

### Zusammensetzungen der Rezepturen 1 und 1 a:

| | Rezeptur 1 | Rezeptur 1a |
|---|---|---|
| MAK HBV | 2,0 mg | 8,0 mg |
| Kaliumphosphatpuffer | 15 mM | 15 mM |
| Natriumchlorid | 30 mM | 30 mM |
| Saccharose | 68,0 mg | 58,0 mg |
| Arginin | 10,0 mg | 10,0 mg |
| Phosphorsäure | ad pH 6,5 | ad pH 6,5 |
| Tween 20 | 0,1 mg | 0,1 mg |
| Wasser für Injektionszwecke | ad 1,0 ml | ad 1,0 ml |

**Tabelle 3a**

| Stabilitätsdaten Rezeptur 1 und Rezeptur 1a bei 25°C | | | | | | |
|---|---|---|---|---|---|---|
| | Lagerung 4 Wochen bei 25°C | | | Lagerung 13 Wochen bei 25°C | | |
| | I | II | III | I | II | III |
| Rez. 1: 2 mg/1 ml | 100 | n.n. | 1,7 | 100 | n.n. | 1,6 |
| Rez. 1a: 8 mg/1 ml | >99 | n.n. | 4,8 | >99 | n.n. | 4,7 |

**Tabelle 3 b**

| Stabilitätsdaten Rezepturen 1 und 1a bei 50°C | | | | | | |
|---|---|---|---|---|---|---|
| | Lagerung 4 Wochen bei 50°C | | | Lagerung 13 Wochen bei 50°C | | |
| | I | II | III | I | II | III |
| Rez. 1: 2 mg/1 ml | >99 | n.n. | 2,0 | >99 | n.n. | 2,0 |
| Rez. 1a: 8 mg/1 ml | >99 | n.n. | 4,7 | >99 | n.n. | 5,5 |
| I Proteingehalt in % mit OD 280 II Aggregate in % mit SE-HPLC III Trübung der rekonstituierten Lösung in Turbiditätseinheiten (dimensionslose Zahl) | | | | | | |

### Beispiel 3

Vergleich der Rezepturen 1 und 4. Rezeptur 4 enthält nur Saccharose als Gerüstbildner, kein Argininphosphat und kein Tween 20. Rezeptur 4 ist instabil.

| | Rezeptur 1 | Rezeptur 4 |
|---|---|---|
| MAK HBV | 2,0 mg | 2,0 mg |
| Kaliumphosphatpuffer | 15 mM | 15 mM |
| Natriumchlorid | 30 mM | 30 mM |
| Saccharose | 68,0 mg | 68,0 mg |
| Arginin | 10,0 mg | -- |
| Phosphorsäure oder KOH | ad pH 6,5 | ad pH 6,5 |
| Tween 20 | 0,1 mg | -- |
| Wasser für Injektionszwecke | ad 1,0 ml | ad 1,0 ml |

**Tabelle 4a**

| Lagerung bei 25°C | | | | | | |
|---|---|---|---|---|---|---|
| | Lagerung 4 Wochen bei 25°C | | | Lagerung 13 Wochen bei 25°C | | |
| | I | II | III | I | II | III |
| Rez. 1: Sacch mit Arg.phos. u. Tween 20 | 100 | n.n. | 1,7 | >99 | n.n. | 1,6 |
| Rez. 4: Sacch ohne Arg. phos. u. Tween 20 | 98,3 | 1,6 | 6,1 | 96,0 | 4,3 | 9,5 |

**Tabelle 4b**

| Lagerung bei 50°C | | | | | | |
|---|---|---|---|---|---|---|
| | Lagerung 4 Wochen bei 50°C | | | Lagerung 13 Wochen bei 50°C | | |
| | I | II | III | I | II | III |
| Rez. 1: Sacch mit Arg.phos. u. Tween 20 | 100 | n.n. | 2,0 | >99 | n.n. | 2,0 |
| Rez. 4: Sacch ohne Arg.phos. und Tween 20 | 96,0 | 4,2 | 8,5 | 89,8 | 10,1 | 10,9 |
| Legende: I Proteingehalt in % mit OD 280 II Aggregate in % mit SE-HPLCIII Trübung der rekonstituierten Lösung in Turbiditätseinheiten (dimensionslose Zahl) III Trübung der rekonstituierten Lösung in Turbiditätseinheiten (dimensionslose Zahl) | | | | | | |

### Beispiel 4

Variation der Aminosäurekomponente der Rezeptur. Die Rezepturen mit basischen, sauren und neutralen Aminosäuren sind stabil.

### Zusammensetzung der Rezepturen:

| | |
|---|---|
| MAK HBV | 2,0 mg |
| Kaliumphosphatpuffer | 15 mM |
| Natriumchlorid | 30 mM |
| Saccharose | 35 - 70 mg |
| Aminosäure | variabel |
| Phosphorsäure oder KOH | ad pH 6,5 |
| Tween 20 | 0,1 mg |
| Wasser für Injektionszwecke | ad 1,0 ml |

**Tabelle 5**

| | Aminosäure |
|---|---|
| Rezeptur 1 | Arginin (basisch) |
| Rezeptur 5 | Ornithin (basisch) |
| Rezeptur 6 | Leucin (neutral) |
| Rezeptur 7 | Asparaginsäure (sauer) |

Der pH-Wert wird durch Phosphorsäure oder Kaliumhydroxidlösung eingestellt.

### Tabellen 6a - d

Untersuchungsergebnisse der Rezepturen 1, 5, 6, 7 nach Lagerung von 4 und 13 Wochen.
a)

**Tabelle 6a**

| Rezeptur 1 (Arginin): | | | | |
|---|---|---|---|---|
| | Lagerzeit 4 Wochen | | Lagerzeit 13 Wochen | |
| | 25°C | 50°C | 25°C | 50°C |
| Proteingehalt % (OD 280) | 100 | >99 | 100 | >99 |
| Aggregate % (SE-HPLC) | n.n. | n.n. | n.n. | n.n. |
| Trübung(Turbidität) | 1,7 | 2,0 | 1,6 | 2,0 |

b)

**Tabelle 6b**

| Rezeptur 5 (Ornithin): | | | | |
|---|---|---|---|---|
| | Lagerzeit 4 Wochen | | Lagerzeit 13 Wochen | |
| | 25°C | 50°C | 25°C | 50°C |
| Proteingehalt % (OD 280) | >99 | >98 | >98 | >98 |
| Aggregate % (SE-HPLC) | n.n. | n.n. | n.n. | n.n. |
| Trübung (Turbidität) | 1,9 | 1,9 | 2,0 | 2,1 |

c)

**Tabelle 6c**

| Rezeptur 6 (Leucin): | | | | |
|---|---|---|---|---|
| | Lagerzeit 4 Wochen | | Lagerzeit 13 Wochen | |
| | 25°C | 50°C | 25°C | 50°C |
| Proteingehalt % (OD 280) | >98 | >98 | >98 | >98 |
| Aggregate % (SE-HPLC) | n.n. | n.n. | 0,1 | 0,1 |
| Trübung (Turbidität) | 2,2 | 2,4 | 2,8 | 2,7 |

d)

**Tabelle 6d**

| Rezeptur 7 (Asparaginsäure): | | | | |
|---|---|---|---|---|
| | Lagerzeit 4 Wochen | | Lagerzeit 13 Wochen | |
| | 25°C | 50°C | 25°C | 50°C |
| Proteingehalt % (OD 280) | > 98 | >98 | >98 | >98 |
| Aggregate % (SE-HPLC) | n.n. | n.n. | 0,1 | 0,1 |
| Trübung (Turbidität) | 2,7 | 2,7 | 3,4 | 4,0 |

### Beispiel 5

Beispiel 5 enthält die Rezeptur 1 bei verschiedenen pH-Werten, die Herstellung der Lyophilisate erfolgte wie in Beispiel 2 beschrieben, die Einstellung des pH der Hilfsstofflösung bzw. der Produktlösung vor Lyophilisation erfolgte mit 85%-iger Phosphorsäure.

### Rezeptur:

| | |
|---|---|
| MAK HBV | 2,0 mg |
| Kaliumphosphatpuffer | 15 mM |
| Natriumchlorid | 30 mM |
| Saccharose | 68 mg |
| Arginin | 10 mg |
| Phosphorsäure | ad pH 5; 6,5; 8 |
| Tween 20 | 0,1 mg |
| Wasser für Injektionszwecke | ad 1,0 ml |

Hergestellt wurden die Lyophilisate mit den pH-Werten aus Tabelle 7.

Nach Verbördelung der Injektionsflaschen wurden diese unter Lichtausschluß bei definierten Temperaturbedingungen eingelagert. Nach Lagerzeiten von 4 Wochen und 13 Wochen wurden die Muster analysiert (Proteingehalt in %: OD 280, Aggregate in %: SE-HPLC, Trübung: Turbidität). Die Rezeptur war bei allen pH-Werten stabil.

**Tabelle 7**

| | pH |
|---|---|
| Rezeptur 8 | 5 |
| Rezeptur 9 (ident. mit 1) | 6,5 |
| Rezeptur 10 | 8 |

**Tabelle 8a**

| Lagerung bei 25°C | | | | | | |
|---|---|---|---|---|---|---|
| | Lagerung 4 Wochen bei 25°C | | | Lagerung 13 Wochen bei 25°C | | |
| | I | II | III | I | II | III |
| Rezeptur 8 | 100 | n.n. | 1,9 | >99 | n.n. | 2,3 |
| Rezeptur 9 (=1) | 100 | n.n | 1,7 | 100 | n.n. | 1,6 |
| Rezeptur 10 | >99 | n.n. | 2,3 | >99 | n.n. | 2,6 |

**Tabelle 8b**

| Lagerung bei 50°C | | | | | | |
|---|---|---|---|---|---|---|
| | Lagerung 13 Wochen bei 50°C | | | Lagerung 13 Wochen bei 50°C | | |
| | I | II | III | I | II | III |
| Rezeptur 8 | >99 | n.n. | 2,2 | >99 | n.n. | 2,3 |
| Rezeptur 9 (=1) | >99 | n.n. | 2,0 | >99 | n.n. | 2,0 |
| Rezeptur 10 | >98 | n.n. | 2,5 | >98 | n.n. | 2,6 |
| Legende: I Proteingehalt in % OD 280 II Aggregate in % mit SE-HPLC III Trübung der rekonstituierten Lösung in Turbiditätseinheiten (dimensionslose Zahl) | | | | | | |

### Beispiel 6

Die nachfolgend beschriebene Rezeptur mit dem Tensid Pluronic F 68 an Stelle von Tween 20 wurde wie zuvor beschrieben hergestellt:

Die Lagerung und Stabilitätsprüfung erfolgt in analoger Weise wie bei den anderen Beispielen.

### Rezeptur 11:

| | |
|---|---|
| MAK HBV | 2,0 mg |
| Kaliumphosphatpuffer | 15 mM |
| Natriumchlorid | 30 mM |
| Saccharose | 48,0 mg |
| Arginin | 10,0 mg |
| Phosphorsäure | ad pH 6,5 |
| Pluronic F 68 | 0,1 mg |
| Wasser für Injektionszwecke | ad 1,0 ml |

Als Vergleich ist Rezeptur 1 gewählt, die mit Rezeptur 11 identisch ist bis auf Tween 20 an Stelle von Pluronic F 68. Beide Rezepturen waren stabil.

**Tabelle 9**

| Stabilitätsdaten der Rezeptur mit den Tensiden Pluronic F 68 und Tween 20. | | | | | | | | |
|---|---|---|---|---|---|---|---|---|
| | Rezeptur 11 | | | | Rezeptur 1 | | | |
| | Lagerzeit 4 Wochen | | Lagerzeit 13 Wochen | | Lagerzeit 4 Wochen | | Lagerzeit 13 Wochen | |
| | 25°C | 50°C | 25°C | 50°C | 25°C | 50°C | 25°C | 50°C |
| Proteingehalt % (OD 280) | >98 | >98 | >98 | >98 | 100 | >99 | 100 | >99 |
| Aggregate % (SE-HPLC) | n.n. | n.n. | n.n. | n.n. | n.n. | n.n. | n.n. | n.n. |
| Trübung (Turbidität) | 1,9 | 1,9 | 2,5 | 2,2 | 1,7 | 2,0 | 1,6 | 2,0 |

### Beispiel 7:

Die in diesem Beispiel beschriebene Rezeptur 12 entspricht im wesentlichen Rezeptur 1, nur wurde hier als Gerüstbildner Mannit an Stelle von Saccharose eingesetzt. Eine Instabilität der Mannitrezeptur ist erkennbar.

### Rezeptur 13

| | |
|---|---|
| MAK HBV | 2,0 mg |
| Kaliumphosphatpuffer | 15 mM |
| Natriumchlorid | 30 mM |
| Mannit | 25,0 mg |
| Arginin | 10,0 mg |
| Phosphorsäure | ad pH 6,5 |
| Tween 20 | 0,1 mg |
| Wasser für Injektionszwecke | ad 1,0 ml |

**Tabelle 10**

| Stabilitätsdaten der Rezepturen mit den Gerüstbildnern Mannit (Rezeptur 12) und Saccharose (Rezeptur 1) | | | | | | | | |
|---|---|---|---|---|---|---|---|---|
| | Rezeptur 12 | | | | Rezeptur 1 | | | |
| | Lagerzeit 4 Wochen | | Lagerzeit 13 Wochen | | Lagerzeit 4 Wochen | | Lagerzeit 13 Wochen | |
| | 25°C | 50°C | 25°C | 50°C | 25°C | 50°C | 25°C | 50°C |
| Proteingehalt % (OD 280) | 96,2 | 91,8 | 94,0 | 84,5 | 100 | >99 | 100 | >99 |
| Aggregate % (SE-HPLC) | 3,6 | 8,4 | 5,8 | 15,9 | n.n. | n.n. | n.n. | n.n. |
| Trübung (Turbidität) | 3,2 | 6,9 | 4,9 | 13,2 | 1,7 | 2,0 | 1,6 | 2,0 |

### Beispiel 8

Ein weiterer Beweis für die Notwendigkeit der Kombination aus Zucker, Aminosäure und Tensid ergibt sich aus dem Vergleich der Rezeptur 1, welche alle aufgeführten Komponenten enthält mit einer Rezeptur 13 aus Antikörper, Kaliumphosphatpuffer, Natriumchlorid und Argininphosphat Ohne Zucker und Tensid ist die Aggregatbildung erhöht und die Trübungswerte werden schlechter.

### Rezeptur 14

| | |
|---|---|
| MAK HBV | 2,0 mg |
| Kaliumphosphatpuffer | 15 mM |
| Natriumchlorid | 30 mM |
| Arginin | 35,0 mg |
| Phosphorsäure | ad pH 6,5 |
| Wasser für Injektionszwecke | ad 1,0 ml |

**Tabelle 11**

| Stabilitätsdaten der Rezeptur 13 (ohne Saccharose und Tween 20, nur mit Argininphosphat als Gerüstbildner) und der Rezeptur 1 | | | | | | | | |
|---|---|---|---|---|---|---|---|---|
| | Rezeptur 14 | | | | Rezeptur 1 | | | |
| | Lagerzeit 4 Wochen | | Lagerzeit 13 Wochen | | Lagerzeit 4 Wochen | | Lagerzeit 13 Wochen | |
| | 25°C | 50°C | 25°C | 50°C | 25°C | 50°C | 25°C | 50°C |
| Proteingehalt %, (OD 280) | 97,6 | 94,9 | 95,8 | 89,0 | 100 | >99 | 100 | >99 |
| Aggregate % (SE-HPLC) | 2,6 | 4,5 | 4,0 | 10,7 | n.n. | n.n. | n.n. | n.n. |
| Trübung Turbidität) | 2,9 | 4,5 | 3,8 | 12,3 | 1,7 | 2,0 | 1,6 | 2,0 |

### Beispiel 9

Ohne Tensid (Tween 20), nur mit Saccharose und Arginin, erhält man zwar eine stabile Rezeptur, die Trübungswerte verschlechtern sich jedoch (Rezeptur 14).

### Rezeptur 14:

| | |
|---|---|
| MAK HBV | 2,0 mg |
| Kaliumphosphatpuffer | 15 mM |
| Natriumchlorid | 30 mM |
| Saccharose | 68,0 mg |
| Arginin | 10,0 mg |
| Phosphorsäure | ad pH 6,5 |
| Wasser für Injektionszwecke | ad 1,0 ml |

**Tabelle 12**

| Stabilitätsdaten der Rezeptur 14 und Rezeptur 1 | | | | | | | | |
|---|---|---|---|---|---|---|---|---|
| | Rezeptur 14 | | | | Rezeptur 1 | | | |
| | Lagerzeit 4 Wochen | | Lagerzeit 13 Wochen | | Lagerzeit 4 Wochen | | Lagerzeit 13 Wochen | |
| | 25°C | 50°C | 25°C | 50°C | 25°C | 50°C | 25°C | 50°C |
| Proteingehalt %, (OD 280) | >99 | >98 | >98 | >98 | 100 | >99 | 100 | >99 |
| Aggregate % (SE-HPLC) | 0,2 | 0,3 | 0,5 | 1,3 | n.n. | n.n. | n.n. | n.n. |
| Trübung (Turbidität) | 3,4 | 4,8 | 8,8 | 13,3 | 1,7 | 2,0 | 1,6 | 2,0 |

### Beispiel 10

Aus der folgenden Zusammenstellung sind die Bestandteile der Rezeptur 15 zu entnehmen Bei dem verwendeten Antikörper handelt es sich um Anti-L-Selektin. Aus den in Tabelle 13 a angegebenen Daten zur Untersuchung der Stabilität geht hervor, daß die verwendete Rezeptur eine hinreichend gute Stabilisierung ermöglicht.

### Zusammensetzungen der Rezeptur 15:

| | Rezeptur 1 |
|---|---|
| Anti-L-Selektin | 7,0 mg |
| Kaliumphosphatpuffer | 15 mM |
| Natriumchlorid | 30 mM |
| Saccharose | 68,0 mg |
| Arginin | 10,0 mg |
| Phosphorsäure | ad pH 6,5 |
| Tween 20 | 0,1 mg |
| Wasser für Injektionszwecke | ad 1,0 ml |

**Tabelle 13 a**

| Stabilitätsdaten Rezeptur 15 bei 25°C | | | | | | |
|---|---|---|---|---|---|---|
| | Lagerung 4 Wochen bei 25°C | | | Lagerung 13 Wochen bei 25°C | | |
| | I | II | III | I | II | III |
| Rez. 15: 7 mg/1 ml | >99 | n.n. | 2,5 | >99 | n.n. | 2,9 |

**Tabelle 13 b**

| Stabilitätsdaten Rezeptur 15 bei 50°C | | | | | | |
|---|---|---|---|---|---|---|
| | Lagerung 4 Wochen bei 50°C | | | Lagerung 13 Wochen bei 50°C | | |
| | I | II | III | I | II | III |
| Rez. 15: 7 mg/1 ml | 99 | n.n. | 4,1 | 99 | n.n. | 5,2 |
| I Proteingehalt in % mit OD 280 II Aggregate in % mit SE-HPLC III Trübung der rekonstituierten Lösung in Turbiditätseinheiten (dimensionslose Zahl) | | | | | | |

## Patentansprüche

1. Stabile lyophilisierte pharmazeutische Zubereitung von mono- oder polyklonalen Antikörpern enthaltend einen Zucker oder einen Aminozucker, eine Aminosäure und ein Tensid.

2. Zubereitung nach Anspruch 1, dadurch gekennzeichnet, daß die Zubereitung im wesentlichen frei von Polyethylenglykolen und/oder frei von proteinartigen pharmazeutisch üblichen Hilfsstoffen ist.

3. Zubereitung nach Anspruch 1 oder 2, dadurch gekennzeichnet, daß der Zucker ein Mono-, Di- oder Trisaccharid ist, vorzugsweise Saccharose, Maltose, Trehalose oder Raffinose.

4. Zubereitung nach einem der Ansprüche 1 - 3, dadurch gekennzeichnet, daß der Aminozucker Glucosamin, N-Methylglucosamin, Galactosamin oder Neuraminsäure ist.

5. Zubereitung nach einem der Ansprüche 1 bis 4, dadurch gekennzeichnet daß die Aminosäure eine basische, saure oder neutrale Aminosäure ist, vorzugsweise Arginin, Lysin, Histidin, Ornithin, Isoleucin, Leucin, Alanin, Glutaminsäure oder Asparaginsäure.

6. Zubereitung nach einem der Ansprüche 1 bis 5, dadurch gekennzeichnet, daß das Tensid ein Polysorbat oder ein Polyoxyethylen-polyoxypropylen-Polymer ist.

7. Zubereitung nach einem der Ansprüche 1 bis 6, dadurch gekennzeichnet, daß sie physiologisch verträgliche Hilfsstoffe aus der Gruppe der Säuren, Basen, Puffer und/oder Isotonisierungsmittel enthält.

8. Wäßrige pharmazeutische Zubereitung von mono- oder polyklonalen Antikörpern erhältlich durch Redissolution des Lyophilisats nach einem der Ansprüche 1 bis 7.

9. Wäßrige pharmazeutische Zubereitung nach Anspruch 8, dadurch gekennzeichnet, daß die Lösung einen pH-Wert von 5 - 8, vorzugsweise von 6 - 7,4 aufweist.

10. Verfahren zur Herstellung einer lyophilisierten pharmazeutischen Zubereitung gemäß einem der Ansprüche 1 bis 7, dadurch gekennzeichnet, daß man eine wäßrige Zubereitung, enthaltend einen monoklonalen oder polyklonalen Antikörper als Wirkstoff und einen Zucker oder Aminozucker, eine Aminosäure und ein Tensid als Zusatzstoffe sowie gegebenenfalls weitere pharmazeutische Hilfsstoffe, herstellt und die Lösung anschließend lyophilisiert.

11. Verwendung eines Zuckers oder Aminozuckers, einer Aminosäure und eines Tensids zur Herstellung stabiler antikörperhaltiger therapeutischer oder diagnostischer Mittel.
